# FASCICULE DE BREVET EUROPEEN

(11) **EP 1 069 151 B1**
(45) Date de publication et mention de la délivrance du brevet: **11.12.2002**
(21) Numéro de dépôt: 00401931.1
(22) Date de dépôt: 05.07.2000
(51) Int. Cl.: C08J 3/09, A61K 7/027

(54) **Composition contenant un polymère organique et un mélange de deux types de solvants volatils et non-volatils**
Organische Polymerzusammensetzung mit gemischten flüchtigen und nichtflüchtigen Lösungsmitteln
Composition containing an organic polymer and mixed volatile and non volatile solvents

(30) Priorité: 16.07.1999 FR 9909265
(43) Date de publication de la demande: 17.01.2001
(73) Titulaire: L'OREAL, 75008 Paris (FR)
(72) Inventeur: Ferrari, Véronique, 94700 Maisons-Alfort (FR); Tournilhac, Florence, 75011 Paris (FR)
(74) Mandataire: Lhoste, Catherine

(56) Documents cités:
- EP-A- 0 195 575
- EP-A- 0 850 644
- US-A- 5 849 275

## Description

La présente invention se rapporte à une composition cosmétique de la peau, y compris du cuir chevelu, et/ou des lèvres des êtres humains, contenant un gel organosoluble notamment réticulé et un mélange de deux solvants volatils et non volatils. Plus spécialement, l'invention se rapporte à une composition de maquillage des matières kératiniques comme la peau, les lèvres et les phanères, conférant un effet de volume.

L'application sur les matières kératiniques d'une composition cosmétique connue conduit à un dépôt qui peut s'affiner au cours du temps, par pénétration dans ces matières de tout ou partie de la composition déposée et/ou par évaporation des constituants volatils présents initialement dans le dépôt. Ces phénomènes peuvent alors révéler les défauts du support comme les ridules, les défauts de pigmentation comme les taches sur les mains et le visage ou la perte de couleur des lèvres, la couperose, ce qui est particulièrement gênant pour un dépôt de fond de teint sur le visage d'êtres humains. En effet, l'un des buts d'un fond de teint est de camoufler les imperfections (taches, points noirs) de la peau et d'en uniformiser le teint.

Par ailleurs, les femmes cherchent de plus en plus à se remodeler le visage et/ou le corps et en particulier les lèvres du visage en vue d'en augmenter leur volume. A ce jour, l'augmentation de volume de certaines parties du visage ou du corps est obtenue par injection de substance telle que des gels de silicone. Ce type de remodelage se fait généralement sous anesthésie locale, voire générale, ce qui peut perturber de façon importante l'organisme. En outre, ce type de remodelage est long, fastidieux et coûteux.

L'invention a justement pour objet une composition cosmétique notamment de maquillage des matières kératiniques comme la peau et/ou les lèvres et/ou les phanères permettant de remédier à ces inconvénients.

Le demandeur a trouvé de manière surprenante que l'association d'un polymère organique avec un mélange de solvants volatils et non volatils permettait un dépôt sur les matières kératiniques, dont le volume augmente au cours du temps sans intervention de stimulus extérieur comme notamment l'humidification du dépôt, permettant de maintenir camouflés durablement les défauts d'aspect des matières kératiniques (taches, points noirs, cernes, plis, creux, minceur).

De façon plus précise, l'invention a pour objet une composition notamment cosmétique contenant au moins un polymère organique, au moins un premier solvant volatil non compatible avec le polymère organique et un second solvant non volatil compatible avec le polymère organique.

Par "solvant volatil", on doit comprendre au sens de l'invention un milieu liquide à température ambiante (25°C°) et pression atmosphérique (76 mm de Hg), aqueux ou non, susceptible de s'évaporer totalement des matières kératiniques. On choisit ces solvants parmi les composés ayant notamment une pression vapeur non nulle, en particulier allant de 10⁻³ à 300 mm de Hg (à température ambiante et pression atmosphérique).

Un "solvant non volatil" est au sens de l'invention un milieu liquide non aqueux à température ambiante et pression atmosphérique, et en particulier un corps gras liquide (appelé aussi huile) ne s'évaporant pas des matières kératiniques. En particulier, il présente une pression de vapeur supérieure à 300mm de Hg (à température ambiante et pression atmosphérique). Selon l'invention, il est possible d'utiliser un ou plusieurs solvants volatils et un ou plusieurs solvants non volatils.

Selon l'invention, les solvants non volatils doivent être compatibles avec le polymère, à savoir capables de le solubiliser à température ambiante. En particulier, le polymère est un gélifiant de ces solvants non volatils. Inversement, les solvants volatils de la composition de l'invention sont incompatibles avec le polymère, et notamment non capables de le solubiliser : autrement dit, le polymère n'est pas un gélifiant de ses solvants volatils. Les solvants volatils et non volatils de l'invention sont tels qu'ils permettent le gonflement du polymère par solubilisation de ce dernier dans les solvants non volatils au fur et à mesure que les solvants volatils s'évaporent du support sur lequel est déposée la composition. Au fur et à mesure de l'évaporation des solvants volatils, le polymère passe de l'état insoluble à l'état soluble et déploie ces chaînes grasses et forme un réseau d'enchevêtrements ou réticulé emprisonnant les solvants non volatils.

Or, dans le document US 5,849,275, les polymères du type silicone/méthacrylique décrits sont solubles dans les solvants volatils indiqués, et sont donc compatibles entre eux. En outre, après évaporation de ces solvants volatils, les polymères précipitent dans les huiles non volatiles du type paraffine ou lanoline, ce qui correspond à une insolubilité de ces polymères dans ces huiles non volatiles. C'est cette précipitation qui confère à la composition décrite des propriétés de non transfert.

Par ailleurs, dans le document EP 0 850 644, il est indiqué que les huiles présentes dans la composition ne doivent pas être volatiles et qu'elles sont piégées dans l'organopolysiloxane. En effet, ces huiles doivent rester sur la peau pour la traiter. Ainsi l'organopolysiloxane est compatible avec les huiles ou les solvants non volatiles. Il est aussi compatible avec les huiles volatiles citées.

Comme solvant volatil utilisable dans l'invention, on peut citer l'eau, les huiles hydrocarbonées volatiles, les huiles siliconées volatiles comportant éventuellement des groupements alkyle ou alkoxy pendants ou en bout de chaîne siliconée et les huiles fluorées volatiles et leurs mélanges. De préférence, on utilise des solvants volatils apolaires.

Comme huile volatile utilisable dans l'invention, on peut citer les silicones linéaires ou cycliques ayant de 2 à 7 atomes de silicium, ces silicones comportant éventuellement des groupes alkyle ou alcoxy ayant de 1 à 10 atomes de carbone pendante ou en bout de chaîne, les isoparaffines en C₈ à C₁₆, les huiles hydrocarbonées perfluorées en C₅ à C₈. Comme huile volatile utilisable dans l'invention, on peut citer notamment l'octaméthylcyclotétra-siloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, l'octaméthyltétrasiloxane ; les isoparaffines en C₈ à C₁₆ telles que les 'ISOPARs', les PERMETYLs et notamment l'isododécane ; les perfluoropolyéthers ayant de 5 à 8 atomes de carbone comme le nonafluorométhoxybutane, le nonafluoroéthoxybutane, le perfluorométhylcyclopentane, le dodécafluoropentané ; leurs mélanges.

La quantité de solvant volatil dépend de l'ampleur du phénomène d'effet volume recherché. En pratique, elle représente notamment de 0,1 à 99,3 % du poids total de la composition, de préférence de 10 à 80 % et mieux de 20 à 75 %.

Comme solvant non volatil utilisable dans l'invention, on peut citer des huiles polaires comme :
- les huiles hydrocarbonées d'origine animale telles que le perhydrosqualène ;
- les huiles hydrocarbonées végétales telles que les triglycérides liquides d'acides gras et de glycérol dont les acides gras peuvent avoir des longueurs de chaînes variées, ces dernières pouvant être linéaires ou ramifiées, saturées ou insaturées: ces huiles sont notamment les huiles de germe de blé, de tournesol, de maïs, de soja, de courge, de pépins de raisin ou de cassis, de sésame, de noisette, d'abricot, de macadamia, de ricin, d'avocat de karité, d'amandes douces, de coton, de luzerne, de pavot, de potimarron, d'onagre, de millet, d'orge, de quinoa, d'olive, de seigle, de carthame, de bancoulier, de passiflore, de rosier muscat ou encore les triglycérides des acides caprylique/caprique comme ceux vendus par la société Stearineries Dubois ou ceux vendus sous les dénominations Miglyol 810, 812 et 818 par la société Dynamit Nobel ;
- les esters naturels ou de synthèse de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone comme par exemple l'huile de Purcellin (octanoate de cétostéaryle), le myristate d'isopropyle, des octanoates, décanoates ou ricinoléates d'alcools ou de polyalcools ;
- les éthers de synthèse de formule R³COR⁴ dans laquelle R³ est un radical alkyle en C₃ à C₁₉ et R⁴ un radical alkyle en C₃ à C₂₀;
- les alcools gras ayant au moins 12 atomes de carbone comme l'octyl dodécanol ou l'alcool oléique ;
- les hydrocarbures cycliques comme les cycloalcanes ou alkylcycloalcanes dont la chaîne alkyle est linéaire ou ramifiée, saturée ou non ayant de 1 à 30 atomes de carbone tels que le cyclohexane, le dioctylcyclohexane ;
- les hydrocarbures aromatiques et notamment les alcènes comme le benzène, le toluène, le 2,4-diméthyl-3-cyclohexène, le dipentène, le p-cymène, le naphtalène, l'anthracène, les esters comme le benzoate d'isostéaryle ;
- les amines primaires, secondaires ou tertiaires comme la triéthanolamine ;
- leurs mélanges.

De préférence, on utilise des esters synthétiques comme le myristate d'isopropyle.

La quantité de ces solvants non volatils est fonction de la quantité de polymère utilisée et de l'ampleur de l'effet de volume recherché. En pratique le ou les solvants non volatils représentent de 0,2 à 99,4 % du poids total de la composition, de préférence de 5 à 90 % et mieux de 20 à 85 %

II est éventuellement possible d'ajouter aux solvants non volatils ci-dessus polaires des huiles apolaires non volatiles, les solvants non volatils étant en particulier, des solvants de ces huiles apolaires comme les huiles siliconées du type polydiméthylsiloxane liquide à température ambiante, les phényldiméthicones, les phényltriméthicones, les polyméthylphényl-siloxanes, les alkylpolydiméthylsiloxanes avec une chaîne alkyle en C₂ à C₂₀ et leurs mélanges; les hydrocarbures linéaires ou ramifiés d'origine synthétique ou minérale comme les huiles de paraffine non volatiles et leurs dérivés, la vaseline, les polydécènes, le polyisobutène hydrogéné tel que le parléam et leur mélange. Ces huiles apolaires peuvent éventuellement être utilisées comme seul solvant non volatil ou mélange de solvants non volatils.

Les huiles non volatiles apolaires doivent être solubilisées par les solvants non volatils et l'ensemble peut représenter de 0 à 80 % du poids total de la composition et mieux de 30 à 80 %.

La nature des solvants non volatils ainsi que celle des solvants volatils est fonction du polymère utilisé. En particulier, lorsque le polymère est un polydiméthylsiloxane réticulé tel que ceux décrits dans le document EP-A-0850644 et en particulier ceux vendus sous la marque KSG 6 ou 16 par la Société Shin Etsu ou Tréfil E-505C ou 506C par la Société Dow Corning, on peut utiliser comme solvants non volatils, les huiles apolaires de silicone mentionnées ci-dessus et comme solvant volatil des huiles hydrocarbonées volatiles comme les isoparaffines en C₈ à C₁₆ du type Isopar's, Permetyls, et en particulier l'isododécane.

En d'autres termes, les solvants non volatils doivent être de bons solvants du polymère et le solvant volatil un mauvais solvant du polymère.

Le polymère organique est avantageusement un polymère radicalaire, non ionique, appartenant à la famille des matériaux super absorbants d'huile, à savoir pouvant reprendre jusqu'à 50 fois son poids en solvant non volatil. Il est notamment choisi parmi les homopolymères ou copolymères styréniques, les homopolymères ou copolymères acryliques et leurs associations. Plus spécialement, le polymère est un homopolymère ou copolymère d'au moins un monomère choisi parmi le styrène, les alkylstyrènes dont le groupement alkyle, linéaire ou ramifié, comporte de 1 à 10 atomes de carbone, les (méth)acrylates d'alkyle dont le groupe alkyle, linéaire ou ramifié comporte de 1 à 10 atomes de carbone, et leurs associations. De préférence, le polymère est un polymère choisi parmi les styrène/(méth)acrylates d'alkyle, les poly(alkystyrène), les alkylstyrène/acrylate d'alkyle. De préférence, le (méth)acrylate d'alkyle est choisi parmi l'acrylate d'éthyl-2-hexyle, le méthacrylate d'isobutyle. A titre d'exemple de polymères utilisables dans l'invention, on peut citer les copolymères de méthylstyrène/acrylate d'éthyl-2-hexyle, styrène/acrylate d'éthyl-2-hexyle/méthacrylate d'isobutyle, les poly(alkylstyrène).

De préférence le polymère est sous forme réticulée. Comme réticulant, on peut utiliser des monomères comportant au moins 2 liaisons éthyléniques, éventuellement conjuguées. A titre d'exemple on peut citer les polyènes comme le butadiène, le bismaléimide, le bisacrylamide. Ce réticulant est présent en une quantité permettant la réticulation maximale du polymère et notamment à une teneur allant de 0,1 à 5 % en poids de l'ensemble (polymère/réticulant).

De façon avantageuse, le polymère de l'invention présente dans la composition au moins une température de transition vitreuse (Tg) inférieure à 60°C et en particulier allant de -100°C à 60°C. Il peut se présenter sous forme de poudre sèche ayant notamment une granulométrie allant de 5 à 600 µm et mieux de 10 à 450 µm.

Comme exemple de polymère organique réticulé utilisable dans l'invention, on peut citer le les produits commerciaux Pliolite AC3-H (méthylstyrène/acrylate d'éthyl-2-hexyle, de Tg de 58°-64°C, à 100 % de matière active) et AC5-G (styrène/acrylate d'éthyl-2-hexyle/méthacrylate d'isobutyle, de Tg 49-55°C à 100 % de matière active), vendus par la société Goodyear , les produits Imbiber Beads (mélange de kaolin et de poly-(alkylstyrène) de 125-420 µm de granulométrie dans un rapport en poids 1/99) et Polymer 295 (poly-(alkylstyrène) de 10-15 µm de granulométrie, à 100 % de matière sèche) vendus par la société Imbibitive Technologies.

Avantageusement, la quantité de polymères est telle que, après évaporation des solvants volatils et éventuellement pénétration de tout ou partie de la formule dans les matières kératiniques, le dépôt restant sur ces matières kératiniques comporte au moins 5 % du poids total du polymère associé aux solvants et huiles non volatiles. En pratique, ces polymères peuvent représenter, en matière active de 0,5% à 80 % du poids total de la composition et mieux de 5% à 60 %.

L'invention s'applique non seulement aux produits de maquillage de la peau aussi bien du visage que du corps humain, y compris du cuir chevelu et de l'intérieur des paupières, des lèvres et des phanères comme les cils, les sourcils, les cheveux et les ongles des êtres humains, mais aussi aux produits de soin et/ou de traitement de la peau, des lèvres et des phanères.

La composition de l'invention peut se présenter sous forme de pâte, de solide, de crème. Elle peut être une émulsion simple ou multiple notamment huile-dans-eau ou eau-dans-huile, un gel anhydre, solide ou souple. De préférence, elle se présente sous forme d'un gel anhydre. Un gel présente une consistance telle que la composition ne s'écoule pas sous son propre poids et tel que le module élastique G* est supérieur au module visqueux G. Ce gel présente notamment une viscosité supérieure à 1 000 Pascals mesurée à 25°C avec un rhéomètre Haake.

Avantageusement la composition est sous forme solide, et notamment coulé en stick ou en coupelle.

L'invention peut, en outre, se présenter sous forme de shampooing traitant ou non, colorant ou non, de produit après shampooing.

La composition selon l'invention peut se présenter sous la forme d'une composition dermatologique ou de soin de la peau, des lèvres ou des phanères ou sous forme d'une composition de protection solaire ou de démaquillage. Elle se présente alors sous forme non colorée, contenant éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent, crème de jour ou de nuit) ou base de soin des ongles.

La composition de l'invention peut également se présenter sous la forme d'un produit coloré de maquillage de la peau, en particulier un fond de teint, un produit anti-cerne, un eye-liner, un fard à joues ou à paupières ou de maquillage des lèvres comme un rouge ou un brillant à lèvres, présentant éventuellement des propriétés de soin ou de traitement ou encore de maquillage des fibres kératiniques comme les mascaras.

Bien entendu la composition de l'invention doit être cosmétiquement ou dermatologiquement acceptable, à savoir non toxique et susceptible d'être appliquée sur la peau, les phanères ou les lèvres d'êtres humains.

La composition de l'invention peut comprendre avantageusement une matière colorante choisie parmi les colorants hydrosolubles ou liposolubles, les pigments, les nacres et leurs mélanges. Cette matière colorant peut représenter de 0,01 à 50 % du poids total de la composition et mieux de 1 à 40%.

Avantageusement, la composition comprend une phase particulaire, généralement présente à raison de 0 à 60% du poids total de la composition, de préférence de 5 à 35 %, et qui peut comprendre, outre des pigments et/ou des nacres, des charges habituellement utilisés dans les compositions cosmétiques ou dermatologiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition et font notamment partie des agents structurants susceptibles de conduire à une forme solide.

Les pigments peuvent être présents dans la composition à raison de 0,05 à 50 % du poids de la composition finale, et de préférence à raison de 2 à 30 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium (DC Red N°21 ou FDC Yellow N° 6).

Les nacres peuvent être présentes dans la composition à raison de 0 à 30 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 60 % du poids total de la composition, de préférence 0,5 à 20 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Coming) et les microbilles de résine de silicone (Tospearl de Toshiba, par exemple).

Avantageusement, la composition de l'invention peut contenir au moins une cire en vue de la rigidifier. La ou les cires peuvent être hydrocarbonées, fluorées et/ou siliconées et être d'origine végétale, minérale, animale et/ou synthétique. En particulier, les cires présentent une température de fusion supérieure à 45 °C.

Comme cire utilisable dans l'invention, on peut citer des cires hydrocarbonées, siliconées et/ou fluorées, comportant éventuellement des fonctions ester, hydroxyle ou thiol. A titre d'exemple, on peut citer, la lanoline, la cire d'abeilles, la cire de Carnauba ou de Candellila, la paraffine, les cires de lignite ou microcristalline, la cérésine ou l'ozokérite ; les cires synthétiques comme les cires de polyéthylène, les cires de silicones comme les alkyl ou alkoxy-diméticone ayant de 16 à 45 atomes de carbone, les cires de Fischer-Tropsch et leurs mélanges.

La nature et la quantité des cires sont fonction des propriétés mécaniques et de textures recherchées. A titre indicatif, la composition peut contenir de 0 à 50 % en poids de cires, par rapport au poids total de la composition et mieux de 5 à 30 %. Ces cires sont, en outre, des agents structurants de la composition.

La composition de l'invention peut comprendre, en outre, un ou plusieurs additifs usuellement utilisés dans le domaine concerné, tels que les antioxydants, les huiles essentielles, les conservateurs, les neutralisants, les actifs cosmétiques ou dermatologiques comme par exemple des émollients, des hydratants, des vitamines, des acides gras essentiels, des filtres solaires, les gélifiants de phase aqueuse. Ces additifs peuvent être présents dans la composition dans les concentrations usuelles, notamment à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition selon l'invention peut être fabriquée par les procédés connus, généralement utilisés dans le domaine cosmétique ou dermatologique.

L'invention a encore pour objet un procédé cosmétique de soin, de maquillage ou de traitement des matières kératiniques comme la peau, les lèvres ou les phanères comme les cils et les cheveux des êtres humains, comprenant l'application sur les matières kératiniques de la composition notamment cosmétique telle que définie ci-dessus.

L'invention a encore pour objet l'utilisation d'au moins un polymère organique associé à au moins un premier solvant volatil non compatible avec le polymère organique et à un second solvant non volatil compatible avec le polymère organique, dans une composition cosmétique ou pour la fabrication d'une composition physiologiquement acceptable à application topique, pour remodeler le visage et/ou le corps et en particulier les lèvres du visage et/ou augmenter le volume des lèvres du visage et/ou camoufler les imperfections et/ou défauts d'aspect des matières kératiniques et/ ou uniformiser le teint, notamment durablement. L'invention est illustrée plus en détail dans les exemples suivants. Les pourcentages sont donnés en poids.

### Exemple 1 : Rouge à lèvres

- Pliolite AC3-H 9%
- Myristate d'isopropyle 81 %
- Isododécane 10 %

*Préparation :* On ajoute le polymère au mélange d'huiles volatiles et non volatiles à température ambiante, après l'avoir chauffer à 60-80 °C sous bullage d'azote. On obtient un mélange fluide.

Le volume du polymère dans le gel est de 9 % du volume total. Le volume du polymère après évaporation de l'isododécane est de 100 % du volume total du dépôt. En appliquant cette composition sur les lèvres, on observe un gonflement ou un lissage de celles-ci au bout de quelques minutes, c'est-à-dire après évaporation d'une partie au moins de l'isododécane. Cet effet visible à l'oeil en comparaison avec l'application d'un polymère filmogène soluble dans l'isododécane seul, pour lequel on observe un film qui s'amenuise au cours du séchage, comme par exemple les alcanes à longues chaînes ou les polymères dispersés et stabilisés en surface par un stabilisant du type Kraton dans l'isododécane (voir le document EP-A-0749747).

### Exemple 2 : Rouge à lèvres

- Cire 15%
- Pigments 9%
- Pliolite AC3-H 7%
- Myristate d'isopropyle 61 %
- Isododécane 8%

Ce rouge à lèvres est fabriqué comme dans l'exemple 1.

## Revendications

1. Composition contenant au moins un polymère organique, au moins un premier solvant volatil non compatible avec le polymère organique, à savoir incapable de solubiliser ledit polymère, et un second solvant non volatil compatible avec le polymère organique, à savoir capable de solubiliser ledit polymère.

2. Composition selon la revendication 1, **caractérisée en ce que** le solvant volatil est un milieu aqueux ou non dont la pression de vapeur va de 10⁻³ à 300mm de Hg (à 25°C et pression atmosphérique).

3. Composition selon la revendication 1 ou 2, **caractérisée en ce que** le solvant volatil est choisi parmi l'eau, les huiles hydrocarbonées volatiles, les huiles siliconées volatiles comportant éventuellement des groupements alkyle ou alkoxy pendants ou en bout de chaîne siliconée et les huiles fluorées volatiles et leurs mélanges.

4. Composition selon l'une quelconque des revendications précédentes, **caractérisée en ce que** le solvant volatil est un solvant apolaire.

5. Composition selon l'une quelconque des revendications précédentes, **caractérisée en ce que** le solvant volatil est choisi parmi l'eau, les silicones linéaires ou cycliques ayant de 2 à 7 atomes de silicium, ces silicones comportant éventuellement des groupes alkyle ou alcoxy ayant de 1 à 10 atomes de carbone pendants ou en bout de chaîne, les isoparaffines en C₈ à C₁₆, les huiles hydrocarbonées perfluorées en C₆ à C₈.

6. Composition selon l'une quelconque des revendications précédentes, **caractérisée en ce que** le solvant volatil est présent en une quantité allant de 0,1 à 99,3 % du poids total de la composition.

7. Composition selon l'une quelconque des revendications précédentes, **caractérisée en ce que** le solvant volatil est présent en une quantité allant de 10 à 80 % du poids total de la composition.

8. Composition selon l'une quelconque des revendications précédentes, **caractérisée en ce que** le solvant non volatil est polaire.

9. Composition selon l'une quelconque des revendications précédentes, **caractérisée en ce que** le solvant non volatil est un milieu liquide non aqueux à température ambiante (25°C).

10. Composition selon l'une quelconque des revendications précédentes, **caractérisée en ce que** le solvant non volatil est choisi parmi:
- les huiles hydrocarbonées d'origine animale;
- les huiles hydrocarbonées végétales,
- les esters naturels ou de synthèse de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone
- les éthers de synthèse de formule R³COR⁴ dans laquelle R³ est un radical alkyle en C₃ à C₁₉ et R⁴ un radical alkyle en C₃ à C₂₀ ;
- les alcools gras ayant au moins 12 atomes de carbone;
- les hydrocarbures cycliques;
- les hydrocarbures aromatiques et les esters;
- les amines primaires, secondaires ou tertiaires ;
- les huiles siliconées ;
- leurs mélanges.

11. Composition selon la revendication 10, **caractérisée en ce que** les huiles hydrocarbonées végétales sont choisies parmi les triglycérides liquides d'acides gras et de glycérol dont les acides gras ont des longueurs de chaînes variées, ces dernières étant linéaires ou ramifiées, saturées ou insaturées ou encore les triglycérides des acides caprylique/caprique et leurs mélanges.

12. Composition selon la revendication 10, **caractérisée en ce que** les hydrocarbures cycliques sont choisis parmi lescycloalcanes ou alkylcycloalcanes dont la chaîne alkyle est linéaire ou ramifiée, saturée ou non ayant de 1 à 30 atomes de carbone et leurs mélanges.

13. Composition selon l'une quelconque des revendications précédentes, **caractérisée en ce que** le solvant non volatil est choisi parmi :
- le perhydrosqualène ;
- les huiles de germe de blé, de tournesol, de maïs, de soja, de courge, de pépins de raisin ou de cassis, de sésame, de noisette, d'abricot, de macadamia, de ricin, d'avocat de karité, d'amandes douces, de coton, de luzerne, de pavot, de potimarron, d'onagre, de millet, d'orge, de quinoa, d'olive, de seigle, de carthame, de bancoulier, de passiflore, de rosier muscat ;
- l'huile de Purcellin (octanoate de cétostéaryle), le myristate d'isopropyle, les octanoates, décanoates ou ricinoléates d'alcools ou de polyalcools ;
- l'octyl dodécanol ou l'alcool oléique;
- le cyclohexane, le dioctylcyclohexane;
- le benzène, le toluène, le 2,4-diméthyl-3-cyclohexène, le dipentène, le p-cymène, le naphtalène, l'anthracène, le benzoate d'isostéaryle;
- la triéthanolamine ;
- les polydiméthylsiloxanes liquides à température ambiante, les phényldiméthicones, les phényltriméthicones, les polyméthylphényl-siloxanes, les alkylpolydiméthylsiloxanes avec une chaîne alkyle en C₂ à C₂₀ ;
- leurs mélanges.

14. Composition selon l'une quelconque des revendications précédentes, **caractérisée en ce que** le solvant non volatil représente de 0,2 à 99,4 % du poids total de la composition.

15. Composition selon l'une quelconque des revendications précédentes, **caractérisée en ce que** le solvant non volatil représente de 5 à 90 % du poids total de la composition.

16. Composition selon l'une quelconque des revendications précédentes, **caractérisée en ce que** le polymère est sous forme réticulée.

17. Composition selon l'une quelconque des revendications précédentes, **caractérisée en ce que** le polymère est un polymère radicalaire, non ionique, appartenant à la famille des matériaux super absorbants d'huile ou des polydiméthylsiloxanes réticulés.

18. Composition selon l'une quelconque des revendications précédentes, **caractérisée en ce que** le polymère est choisi parmi les homopolymères ou copolymères d'au moins un monomère choisi parmi le styrène, les alkylstyrènes dont le groupement alkyle, linéaire ou ramifié, comporte de 1 à 10 atomes de carbone, les (méth)acrylates d'alkyle dont le groupe alkyle, linéaire ou ramifié comporte de 1 à 10 atomes de carbone, et leurs associations.

19. Composition selon l'une quelconque des revendications précédentes, **caractérisée en ce que** le polymère est choisi parmi les copolymères de méthylstyrène/acrylate d'éthyl-2-hexyle, styrène/acrylate d'éthyl-2-hexyle/méthacrylate d'isobutyle, les poly(alkylstyrène).

20. Composition selon l'une quelconque des revendications précédentes, **caractérisée en ce que** le polymère présente dans la composition au moins une température de transition vitreuse (Tg) inférieure à 60°C.

21. Composition selon l'une quelconque des revendications précédentes, **caractérisée en ce que** le polymère représente, en matière active de 0,5% à 80 % du poids total de la composition.

22. Composition selon l'une quelconque des revendications précédentes, **caractérisée en ce que** le polymère représente, en matière active de 5% à 60 % du poids total de la composition.

23. Composition selon l'une quelconque des revendications précédentes, **caractérisée en ce qu'**elle constitue un produit de soin et/ou de traitement de la peau des lèvres et des phanères, un produit de maquillage de la peau, des lèvres ou des phanères.

24. Composition selon l'une quelconque des revendications précédentes, **caractérisée en ce qu'**elle se présente sous forme d'un gel anhydre.

25. Composition selon l'une quelconque des revendications précédentes, **caractérisée en ce qu'**elle se présente sous forme solide.

26. Composition selon l'une quelconque des revendications précédentes, **caractérisée en ce qu'**elle contient, en outre, un ou plusieurs additifs choisis parmi les matières colorantes, les antioxydants, les huiles essentielles, les conservateurs, les neutralisants, les actifs cosmétiques ou dermatologiques, les gélifiants de phase aqueuse, les charges.

27. Composition selon l'une quelconque des revendications précédentes, **caractérisée en ce qu'**elle contient, en outre, une matière colorante.

28. Composition selon l'une quelconque des revendications précédentes, **caractérisée en ce qu'**elle contient, en outre, au moins une ciré.

29. Procédé de soin, de maquillage ou de traitement cosmétique des matières kératiniques telles que la peau, les lèvres ou les phanères des êtres humains, comprenant l'application sur les matières kératiniques de la composition cosmétique selon l'une quelconque des revendications précédentes.

30. Utilisation d'au moins un polymère organique associé à au moins un premier solvant volatil non compatible avec le polymère organique, à savoir incapable de solubiliser ledit polymère, et à un second solvant non volatil compatible avec le polymère organique, à savoir capable de solubiliser ledit polymère, dans une composition cosmétique ou pour la fabrication d'une composition physiologiquement acceptable à application topique, pour remodeler le visage et/ou le corps et/ou augmenter le volume des lèvres du visage et/ou camoufler les imperfections et/ou défauts d'aspect des matières kératiniques et/ou uniformiser le teint.

## Claims

1. Composition containing at least one organic polymer, at least one first volatile solvent which is incompatible with the organic polymer, that is to say which is incapable of dissolving the said polymer, and one second nonvolatile solvent which is compatible with the organic polymer, that is to say which is capable of dissolving the said polymer.

2. Composition according to Claim 1, **characterized in that** the volatile solvent is an aqueous or nonaqueous medium whose vapour pressure ranges from 10⁻³ mmHg to 300 mmHg (at 25°C and atmospheric pressure).

3. Composition according to Claim 1 or 2, **characterized in that** the volatile solvent is chosen from water, volatile hydrocarbon-based oils, volatile silicone oils optionally comprising alkyl or alkoxy groups which are pendant or at the end of the silicone chain, and volatile fluoro oils, and mixtures thereof.

4. Composition according to any one of the preceding claims, **characterized in that** the volatile solvent is an apolar solvent.

5. Composition according to any one of the preceding claims, **characterized in that** the volatile solvent is chosen from water, linear or cyclic silicones containing from 2 to 7 silicon atoms, these Silicones optionally comprising alkyl or alkoxy groups containing from 1 to 10 carbon atoms that are pendant or at the end of a chain, C₈ to C₁₆ isoparaffins and C₅ to C₈ perfluorohydrocarbon-based oils.

6. Composition according to any one of the preceding claims, **characterized in that** the volatile solvent is present in an amount ranging from 0.1% to 99.3% of the total weight of the composition.

7. Composition according to any one of the preceding claims, **characterized in that** the volatile solvent is present in an amount ranging from 10% to 80% of the total weight of the composition.

8. Composition according to any one of the preceding claims, **characterized in that** the nonvolatile solvent is polar.

9. Composition according to any one of the preceding claims, **characterized in that** the nonvolatile solvent is a nonaqueous medium which is liquid at room temperature (25°C).

10. Composition according to any one of the preceding claims, **characterized in that** the nonvolatile solvent is chosen from:
- hydrocarbon-based oils of animal origin;
- hydrocarbon-based plant oils;
- natural or synthetic esters of formula R₁COOR₂ in which R₁ represents a higher fatty acid residue containing from 7 to 19 carbon atoms and R₂ represents a branched hydrocarbon-based chain containing from 3 to 20 carbon atoms;
- synthetic ethers of formula R³COR⁴ in which R³ is a C₃ to C₁₉ alkyl radical and R⁴ is a C₃ to C₂₀ alkyl radical;
- fatty alcohols containing at least 12 carbon atoms;
- cyclic hydrocarbons;
- aromatic hydrocarbons and esters;
- primary, secondary or tertiary amines;
- silicone oils;
- mixtures thereof.

11. Composition according to Claim 10, **characterized in that** the hydrocarbon-based plant oils are chosen from liquid triglycerides of fatty acids and of glycerol, in which the fatty acids have varied chain lengths, these chains being linear or branched, and saturated or unsaturated, or caprylic/capric acid triglycerides, and mixtures thereof.

12. Composition according to Claim 10, **characterized in that** the cyclic hydrocarbons are chosen from cycloalkanes or alkylcycloalkanes in which the alkyl chain is linear or branched, saturated or unsaturated and contains from 1 to 30 carbon atoms, and mixtures thereof.

13. Composition according to any one of the preceding claims, **characterized in that** the nonvolatile solvent is chosen from:
- perbydrosqualene;
- wheatgerm oil, sunflower oil, corn oil, soybean oil, marrow oil, grapeseed oil, blackcurrant seed oil, sesame oil, hazelnut oil, apricot oil, macadamia oil, castor oil, avocado oil, karite butter, sweet almond oil, cotton oil, alfalfa oil, poppy oil, pumpkin oil, evening primrose oil, millet oil, barley oil, quinoa oil, olive oil, rye oil, safflower oil, candlenut oil, passion flower oil and musk rose oil;
- purcellin oil (cetostearyl octanoate), isopropyl myristate, alkyl or polyalkyl octanoates, decanoates or ricinoleates;
- octyldodecanol and oleyl alcohol;
- cyclohexane and dioctylcyclohexane;
- benzene, toluene, 2,4-dimethyl-3-cyclohexene, dipentene, p-cymene, naphthalene, anthracene or isostearyl benzoate;
- triethanolamine;
- polydimethyl siloxanes that are liquid at room temperature, phenyldimethicones, phenyltrimethicones, polymethylphenylsiloxanes and alkyl polydimethyl siloxanes with a C₂ to C₂₀ alkyl chain;
- mixtures thereof.

14. Composition according to any one of the preceding claims, **characterized in that** the nonvolatile solvent represents from 0.2% to 99.4% of the total weight of the composition.

15. Composition according to any one of the preceding claims, **characterized in that** the nonvolatile solvent represents from 5% to 90% of the total weight of the composition.

16. Composition according to any one of the preceding claims, **characterized in that** the polymer is in crosslinked form.

17. Composition according to any one of the preceding claims, **characterized in that** the polymer is a nonionic radical-mediated polymer belonging to the family of oil-superabsorbent materials ,or crosslinked polydimethylsiloxanes.

18. Composition according to any one of the preceding claims, **characterized in that** the polymer is chosen from homopolymers or copolymers of at least one monomer chosen from styrene, alkylstyrenes in which the linear or branched alkyl group contains from 1 to 10 carbon atoms, and alkyl (meth)acrylates in which the linear or branched alkyl group contains from 1 to 10 carbon atoms, and combinations thereof.

19. Composition according to any one of the preceding claims, **characterized in that** the polymer is chosen from methylstyrene/2-ethylhexyl acrylate and styrene/2-ethylhexyl acrylate/isobutyl methacrylate copolymers and poly(alkylstyrene)s.

20. Composition according to any one of the preceding claims, **characterized in that** the polymer in the composition has at least one glass transition temperature (Tg) of less than 60°C.

21. Composition according to any one of the preceding claims, **characterized in that** the polymer represents, in terms of active material, from 0.5% to 80% of the total weight of the composition.

22. Composition according to any one of the preceding claims, **characterized in that** the polymer represents, in terms of active material, from 5% to 60% of the total weight of the composition.

23. Composition according to any one of the preceding claims, **characterized in that** it constitutes a care and/or treating product for the skin, the lips and superficial body growths, or a make-up product for the skin, the lips or superficial body growths.

24. Composition according to any one of the preceding claims, **characterized in that** it is in the form of an anhydrous gel.

25. Composition according to any one of the preceding claims, **characterized in that** it is in solid form.

26. Composition according to any one of the preceding claims, **characterized in that** it also contains one or more additives chosen from dyestuffs, antioxidants, essential oils, preserving agents, neutralizing agents, cosmetic or dermatological active agents, aqueous-phase gelling agents and fillers.

27. Composition according to any one of the preceding claims, **characterized in that** it also contains a dyestuff.

28. Composition according to any one of the preceding claims, **characterized in that** it also contains at least one wax.

29. Cosmetic process for caring for, making up or treating keratin materials such as the skin, the lips or superficial body growths of human beings, comprising the application of the cosmetic composition according to any one of the preceding claims to the keratin materials.

30. Use of at least one organic polymer combined with at least one first volatile solvent which is incompatible with the organic polymer, that is to say which is incapable of dissolving the said polymer, and with a second nonvolatile solvent which is compatible with the organic polymer, that is to say which is capable of dissolving the said polymer, in a cosmetic composition or for the manufacture of a physiologically acceptable composition for topical application, to remodel the face and/or the body and/or to increase the volume of the lips of the face and/or to camouflage the aesthetic imperfections and/or defects of keratin materials and/or to unify the complexion.

## Patentansprüche

1. Zusammensetzung, die mindestens ein organisches Polymer, mindestens ein erstes flüchtiges Lösemittel, das mit dem organischen Polymer nicht kompatibel ist, d.h. nicht befähigt ist, das Polymer zu solubilisieren, und ein zweites nicht flüchtiges Lösemittel, das mit dem organischen Polymer kompatibel ist, d.h. befähigt ist, das Polymer zu solubilisieren, enthält.

2. Zusammensetzung nach Anspruch 1, **dadurch gekennzeichnet, dass** es sich bei dem flüchtigen Lösemittel um ein wässeriges Medium oder ein nicht wässeriges Medium handelt, dessen Dampfdruck im Bereich von 10⁻³ bis 300 mm Hg liegt (bei 25 °C und Atmosphärendruck).

3. Zusammensetzung nach Anspruch 1 oder 2, **dadurch gekennzeichnet, dass** das flüchtige Lösemittel unter Wasser, flüchtigen Kohlenwasserstoffölen, flüchtigen Siliconölen, die gegebenenfalls Alkylgruppen oder Alkoxygruppen aufweisen, die als Seitenketten oder am Ende der Siliconkette vorliegen, flüchtigen fluorierten Ölen und deren Gemischen ausgewählt ist.

4. Zusammensetzung nach einem der vorhergehenden Ansprüche, **dadurch gekennzeichnet, dass** das flüchtige Lösemittel ein apolares Lösemittel ist.

5. Zusammensetzung nach einem der vorhergehenden Ansprüche, **dadurch gekennzeichnet, dass** das flüchtige Lösemittel unter Wasser, geradkettigen oder cyclischen Siliconen mit 2 bis 7 Siliciumatomen, wobei die Silicone gegebenenfalls Alkylgruppen oder Alkoxygruppen mit 1 bis 10 Kohlenstoffatomen aufweisen, die als Seitenketten oder am Ende der Kette vorliegen, C₈₋₁₆-Isoparaffinen und perfluorierten C₅₋₈-Kohlenwasserstoffölen ausgewählt ist.

6. Zusammensetzung nach einem der vorhergehenden Ansprüche, **dadurch gekennzeichnet, dass** das flüchtige Lösemittel in einem Mengenanteil von 0,1 bis 99,3 % des Gesamtgewichts der Zusammensetzung vorliegt.

7. Zusammensetzung nach einem der vorhergehenden Ansprüche, **dadurch gekennzeichnet, dass** das flüchtige Lösemittel in einem Mengenanteil von 10 bis 80 % des Gesamtgewichts der Zusammensetzung vorliegt.

8. Zusammensetzung nach einem der vorhergehenden Ansprüche, **dadurch gekennzeichnet, dass** das nicht flüchtige Lösemittel polar ist.

9. Zusammensetzung nach einem der vorhergehenden Ansprüche, **dadurch gekennzeichnet, dass** das nicht flüchtige Lösemittel ein bei Raumtemperatur (25 °C) flüssiges, nicht wässeriges Medium ist.

10. Zusammensetzung nach einem der vorhergehenden Ansprüche, **dadurch gekennzeichnet, dass** das nicht flüchtige Lösemittel ausgewählt ist unter:
- Kohlenwasserstoffölen tierischen Ursprungs,
- pflanzlichen Kohlenwasserstoffölen,
- natürlichen oder synthetischen Estern der Formel R₁COOR₂, worin R₁ den Rest einer höheren Fettsäure mit 7 bis 19 Kohlenstoffatomen und R₂ eine verzweigte Kohlenwasserstoffkette mit 3 bis 20 Kohlenstoffatomen bedeuten,
- synthetischen Ethern der Formel R³COR⁴, worin R³ eine C₃₋₁₉-Alkylgruppe und R⁴ eine C₃₋₂₀-Alkylgruppe bedeuten,
- Fettalkoholen mit mindestens 12 Kohlenstoffatomen,
- cyclischen Kohlenwasserstoffen,
- aromatischen Kohlenwasserstoffen und Estern,
- primären, sekundären oder tertiären Aminen,
- Siliconölen und
- den Gemischen dieser Verbindungen.

11. Zusammensetzung nach Anspruch 10, **dadurch gekennzeichnet, dass** die pflanzlichen Kohlenwasserstofföle unter flüssigen Trigiyceriden von Fettsäuren und Glycerin, worin die Fettsäuren unterschiedliche Kettenlängen aufweisen, wobei die Ketten linear oder verzweigt, gesättigt oder ungesättigt sind, oder auch Triglyceriden von Capryl-/Caprinsäure und deren Gemischen ausgewählt sind.

12. Zusammensetzung nach Anspruch 10, **dadurch gekennzeichnet, dass** die cyclischen Kohlenwasserstoffe unter Cycloalkanen oder Alkylcycloalkanen mit geradkettiger oder verzweigter, gesättigter oder ungesättigter Alkylkette mit 1 bis 30 Kohlenstoffatomen und deren Gemischen ausgewählt sind.

13. Zusammensetzung nach einem der vorhergehenden Ansprüche, **dadurch gekennzeichnet, dass** das nicht flüchtige Lösemittel ausgewählt ist unter:
- Perhydrosqualen,
- Weizenkeimöl, Sonnenblumenöl, Maisöl, Sojaöl, Kürbisöl, Traubenkernöl, Johannisbeerkernöl, Sesamöl, Haselnussöl, Aprikosenöl, Macadamiaöl, Ricinusöl, Avocadoöl, Sheaöl, süßem Mandelöl, Baumwollsamenöl, Luzernenöl, Mohnöl, Öl von rotem Hokkaidokürbis (Potimarron), Nachtkerzenöl, Hirseöl, Gerstenöl, Quinoaöl, Olivenöl, Roggenöl, Safloröl, Lichtnussöl, Öl von Passionsblumen und Muskatrosenöl,
- Purcellinöl (Cetostearyloctanoat), Isopropylmyristat und Octanoaten, Decanoaten oder Ricinoleaten von Alkoholen oder Polyalkoholen,
- Octyldodecanol oder Oleylalkohol,
- Cyclohexan und Dioctylcyclohexan,
- Benzol, Toluol, 2,4-Dimethyl-3-cyclohexen, Dipenten, p-Cymol, Naphthalin, Anthracen und Isostearylbenzoat,
- Triethanolamin,
- bei Raumtemperatur flüssigen Polydimethylsiloxanen, Phenyldimeticonen, Phenyltrimeticonen, Polymethylphenylsiloxanen und Alkylpolydimethylsiloxanen mit einer C₂₋₂₀-Alkylkette sowie
- deren Gemischen,

14. Zusammensetzung nach einem der vorhergehenden Ansprüche, **dadurch gekennzeichnet, dass** das nicht flüchtige Lösemittel 0,2 bis 99,4 % des Gesamtgewichts der Zusammensetzung ausmacht.

15. Zusammensetzung nach einem der vorhergehenden Ansprüche, **dadurch gekennzeichnet, dass** das nicht flüchtige Lösemittel 5 bis 90 % des Gesamtgewichts der Zusammensetzung ausmacht.

16. Zusammensetzung nach einem der vorhergehenden Ansprüche, **dadurch gekennzeichnet, dass** das Polymer in vernetzter Form vorliegt.

17. Zusammensetzung nach einem der vorhergehenden Ansprüche, **dadurch gekennzeichnet, dass** das Polymer ein nichtionisches, durch radikalische Polymerisation erhaltenes Polymer ist, das zur Gruppe der Superabsorber für Öl oder der vernetzten Polydimethylsiloxane zählt.

18. Zusammensetzung nach einem der vorhergehenden Ansprüche, **dadurch gekennzeichnet, dass** das Polymer unter Homopolymeren oder Copolymeren mindestens eines Monomers ausgewählt ist, das unter Styrol, Alkylstyrolen, deren geradkettige oder verzweigte Alkylgruppe 1 bis 10 Kohlenstoffatome aufweist, Alkyl(meth)acrylaten, deren geradkettige oder verzweigte Alkylgruppe 1 bis 10 Kohlenstoffatome aufweist, und deren Kombinationen ausgewählt ist.

19. Zusammensetzung nach einem der vorhergehenden Ansprüche, **dadurch gekennzeichnet, dass** das Polymer unter Methylstyro1/2-Ethylhexylacrylat- und Styrol/2-Ethylhexylacrylat/Isobutylmethacrylat-Copolymeren und Poly(alkylstyrolen) ausgewählt ist.

20. Zusammensetzung nach einem der vorhergehenden Ansprüche, **dadurch gekennzeichnet, dass** das Polymer in der Zusammensetzung eine Glasübergangstemperatur (Tg) zumindest unter 60 °C aufweist.

21. Zusammensetzung nach einem der vorhergehenden Ansprüche, **dadurch gekennzeichnet, dass** das Polymer ausgedrückt als Wirkstoff 0,5 bis 80 % des Gesamtgewichts der Zusammensetzung ausmacht.

22. Zusammensetzung nach einem der vorhergehenden Ansprüche, **dadurch gekennzeichnet, dass** das Polymer ausgedrückt als wirkstoff 5 bis 60 % des Gesamtgewichts der Zusammensetzung ausmacht.

23. Zusammensetzung nach einem der vorhergehenden Ansprüche, **dadurch gekennzeichnet, dass** sie ein Produkt zur Pflege und/oder zur Behandlung der Haut, der Lippen und der Hautanhangugebilde oder ein Produkt zum Schminken der Haut, der Lippen oder der Hautanhangsgebilde darstellt.

24. Zusammensetzung nach einem der vorhergehenden Ansprüche, **dadurch gekennzeichnet, dass** sie in Form eines wasserfreien Gels vorliegt.

25. Zusammensetzung nach einem der vorhergehenden Ansprüche, **dadurch gekennzeichnet, dass** sie in fester Form vorliegt.

26. Zusammensetzung nach einem der vorhergehenden Ansprüche, **dadurch gekennzeichnet, dass** sie ferner einen oder mehrere Zusatzstoffe enthält, die unter Farbmitteln, Antioxidantien, etherischen Ölen, Konservierungsstoffen, Neutralisationsmitteln, kosmetischen oder dermatologischen Wirkstoffen, Gelbildnern für wässerige Phasen und Füllstoffen ausgewählt sind.

27. Zusammensetzung nach einem der vorhergehenden Ansprüche, **dadurch gekennzeichnet, dass** sie ferner ein Farbmittel enthält.

28. Zusammensetzung nach einem der vorhergehenden Ansprüche, **dadurch gekennzeichnet, dass** sie ferner mindestens ein Wachs enthält.

29. Verfahren zur Pflege, zum Schminken oder zur kosmetischen Behandlung keratinischer Materialien, wie der Haut, der Lippen oder der Hautanhangsgebilde von Personen, das die Anwendung der kosmetischen Zusammensetzung nach einem der vorhergehenden Ansprüche auf die keratinischen Materialien umfasst.

30. Verwendung mindestens eines organischen Polymers in Kombination mit mindestens einem ersten flüchtigen Lösemittel, das mit dem organischen Polymer nicht kompatibel ist, d.h. nicht befähigt ist, das Polymer zu solubilisieren, und einem zweiten nicht flüchtigen Lösemittel, das mit dem organischen Polymer kompatibel ist, d.h. befähigt ist, das Polymer zu solubilisieren, in einer kosmetischen Zusammensetzung oder zur Herstellung einer physiologisch akzeptablen Zusammensetzung zur topischen Anwendung, um die Form des Gesichts und/oder des Körpers zu verändern und/oder das Volumen der Lippen zu vergrößern und/oder Fehler und/oder Mängel im Erscheinungsbild der keratinischen Materialien zu verbergen und/oder für einen einheitlichen Teint zu sorgen.
